(19) Europäisches Patentamt / European Patent Office / Office européen des brevets

(11) **EP 2 429 588 B1**

(12) **EUROPEAN PATENT SPECIFICATION**

(45) Date of publication and mention
of the grant of the patent:
**18.11.2020 Bulletin 2020/47**

(51) Int Cl.:
***A61K 49/00*** *(2006.01)*

(21) Application number: **10770345.6**

(22) Date of filing: **29.04.2010**

(86) International application number:
**PCT/US2010/032997**

(87) International publication number:
**WO 2010/127136 (04.11.2010 Gazette 2010/44)**

(54) **MEASUREMENT OF BODY FLUID VOLUMES**

MESSUNG VON KÖRPERFLÜSSIGKEITSVOLUMEN

MESURE DE VOLUMES DE FLUIDE CORPOREL

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **30.04.2009 US 174100 P**

(43) Date of publication of application:
**21.03.2012 Bulletin 2012/12**

(73) Proprietor: **Pharmacophotonics, Inc.
Indianapolis, IN 46202 (US)**

(72) Inventors:
• **MOLITORIS, Bruce A.
Indianapolis
IN 46260 (US)**
• **WANG, Exing
Carmel
IN 46032 (US)**
• **SANDOVAL, Ruben M., Jr.
Indianapolis
IN 46220 (US)**

(74) Representative: **Lee, Nicholas John et al
Kilburn & Strode LLP
Lacon London
84 Theobalds Road
London WC1X 8NL (GB)**

(56) References cited:
US-A- 5 685 302    US-A1- 2007 218 563
US-B1- 6 355 624

• D. K. BINDER: "In Vivo Measurement of Brain
Extracellular Space Diffusion by Cortical Surface
Photobleaching", JOURNAL OF
NEUROSCIENCE, vol. 24, no. 37, 15 September
2004 (2004-09-15), pages 8049-8056,
XP055163180, ISSN: 0270-6474, DOI:
10.1523/JNEUROSCI.2294-04.2004
• M.J. DURKOT ET AL: "Chronic hypobaric hypoxia
decreases intracellular and total body water in
microswine", COMPARATIVE BIOCHEMISTRY
AND PHYSIOLOGY PART A: PHYSIOLOGY, vol.
114, no. 2, 1 June 1996 (1996-06-01), pages
117-121, XP055163545, ISSN: 0300-9629, DOI:
10.1016/0300-9629(95)02103-5
• WEIMING YU ET AL: "Rapid determination of
renal filtration function using an optical
ratiometric imaging approach", AJP: RENAL
PHYSIOLOGY, AMERICAN PHYSIOLOGICAL
SOCIETY, vol. 292, no. 6, 1 June 2007
(2007-06-01), pages F1873-F1880, XP002636079,
ISSN: 0363-6127, DOI:
10.1152/AJPRENAL.00218.2006 [retrieved on
2007-02-01]

EP 2 429 588 B1

**Description**

TECHNICAL FIELD

The present invention is related generally to measurement of body fluid volumes in an animal subject. The body fluid volumes of interest include extracellular fluid volume (ECFV), total vascular plasma volume (TVPV) and interstitial fluid volume (IFV). The methods are especially beneficial for subjects suffering from renal failure, and particularly those undergoing renal dialysis.

BACKGROUND OF THE INVENTION

The present invention discloses methods and apparatus for measuring the various body fluid volumes in an animal, particularly in animals with renal failure, and more particularly in a renal dialysis patients. The body fluid volumes of interest in the present application are extracellular fluid volume (ECFV), total vascular plasma volume (TVPV) and interstitial fluid volume (IFV).

Body fluid volume status is a critical metric in the management of many chronic and acute medical conditions. Volume status is a key determinant in drug dosing, pharmacokinetics, blood pressure and organ perfusion. Volume status and volume management are most critical in indications or conditions such as, but are not limited to, end stage renal disease (ESRD), hypertension, congestive heart failure, septic shock and hypovolemia, acute kidney injury and chronic kidney disease (CKD), hypertension, syncope, acute blood loss, pre-surgical screening, orthostatic hypotension and anemia in cancer or HIV. In addition, evaluating total vascular plasma volume and interstitial fluid volume in dialysis patients has very important implications especially with regard to removal of volume while on dialysis. This is clinically very important for control of blood pressure and clinical outcomes in patients with end stage renal disease (ESRD) who all require chronic forms of dialysis or renal replacement therapy (RRT) for volume removal. The importance of volume status and volume management in dialysis patients has been discussed by Agarwal R. et al. ("Diagnostic Utility of Blood Volume Monitoring in Hemodialysis Patients" Am J of Kidney Diseases (2008) 51: 242-254), Rodriguez H. J. et al. ("Assessment of Dry Weight by Monitoring Changes in Blood Volume During Hemodialysis using Crit-Line" Kidney International (2005) 68, 854-861), Kraemer M. et al. ("Detection Limit of Methods to Assess Fluid Status Changes in Dialysis Patients" Kidney International (2006) 69: 1609-1620) and Dasselaar J. J. et al. ("Measurment of Relative Blood Volume Changes During Haemodialysis: Merits and Limitations" Nephrol Dial Transplant (2005) 20: 2043-2049).

A commonly used technique for estimating the TVPV is based on the concept of the indicator dilution technique in which an indicator molecule is mixed and distributed into an unknown volume. An identical amount of the indicator molecule is placed into a known volume. The unknown volume can be measured by comparing the concentration of the indicator between the known and unknown volume. A common indicator molecule that is being used is albumin labeled with various dyes, such as radioactive iodine ($I^{125}$ or $I^{131}$) or the fluorescent dye indocyanine green (ICG). For example, Daxor Corporation (New York, NY) has developed a device for measuring blood volume using albumin labeled with $I^{131}$ as the tracer indicator. Use of ICG-labeled albumin as the tracer indicator has been disclosed by Mitra, S. et al. ("Serial Determinations of Absolute Plasma Volume with Indocyanine Green During Hemodialyais," J Am Soc of Nephrology. (2003) 14(9): 2345-51). In this method, ICG-labeled albumin was measured by near infra-red absorption of the molecule. Functionally, there is little difference between the use of ICG when compared to $I^{131}$, as both quickly bind to albumin in the bloodstream. The main distinguishing characteristics are the relatively short half life of ICG as compared to $I^{131}$ and the beneficial safety profile of ICG. ICG is already approved for human use by the United States Food And Drug Administration (FDA). The short half life of ICG allows for multiple tests to be conducted with rapid succession. However, utility of the ICG method has been limited by many of the same factors as the iodine-based testing. Though the time period for collecting samples of ICG is much shorter than the radioactive test, it becomes all the more important to make certain that sampling is conducted at precise time intervals. Therefore, it is a very labor intensive method. Another drawback in the use of labeled albumin, in the dilution technique to measure plasma volume, is that albumin also "leaks" and distributes to the interstitial fluid. Under physiologic conditions, albumin "leaks" into the interstitial space at a rate of about 5% per hour.

This rate increases to 15% per hour in patients with septic shock (see US Pat. No. 6,355,624). Thus, albumin does not measure the true TVPV or plasma volume, but rather it measures the combination of the TVPV and the IFV.

Weiming et al discloses a ratiometric measurement technique that allows rapid measurement of plasma clearance rates of a particular molecule and glomerular filtration rate (GFR) using intravital microscopy. The measurement technique is based on the use of two fluorescent molecules and the ratio between their intensity values.

Another method that is used to measure body fluid volumes is the use of bioimpedence spectroscopy. This approach has been discussed by Zhu et al. ("Segment-Specific Resistivity Improves Body Fluid Volume Estimates from Bioimpedence Spectroscopy in Hemodialysis Patients" J Appl Physio (2006) 100: 717-724), De Lorenzo A. et al. ("Predicting Body Cell Mass With Bioimpedance by Using Theoretical Methods: a Technological Review" J Appl Physiology (1997) 82: 1542-1558) and Kuhlmann, M. K. et al. ("Bioimpedence, Dry Weight and Blood Pressure Control: New Methods and Consequences" Current Opinion in Nephrology and Hypertension (2005) 14: 543-549). However, this technique is too difficult and impractical to perform.

Therefore, there is a clinical need to develop a minimally invasive method to accurately and inexpensive quantify these body fluid volumes. The present invention is provided to solve the problems discussed above and other problems, and to provide advantages and aspects not provided by prior techniques. A full discussion of the features and advantages of the present invention is deferred to the following detailed description.

SUMMARY OF THE INVENTION

In a first aspect, the invention provides an injectate containing a known amount $A_1$ of a first molecule and a known amount $A_2$ of a second molecule, wherein the first molecule is dextran with a molecular size of 1 kDa to 20 kDa labeled with a first fluorescent dye and is non-metabolized and permeable to vessel walls of the vascular system of the animal, and the second molecule is dextran with a molecular size of 70 kDa to 500 kDa labeled with a second fluorescent dye molecule and is non-metabolized and impermeable to vessel walls of the vascular system of the animal, for use in a method for simultaneously measuring extracellular fluid volume (ECFV) and total vascular plasma volume (TVPV) in an animal with renal failure, the method comprising:

    (a) administering the injectate into the vascular system of the animal;
    (b) allowing the first molecule to reach a first equilibrium steady state concentration $C_1$ and the second molecule to reach a second equilibrium steady state concentration $C_2$;
    (c) measuring $C_1$ and $C_2$ in the vascular system of the animal;
    (d) calculating ECFV using the equation ECFV = $A_1/C_1$ and TVPV using the equation TVPV = $A_2/C_2$, and optionally
    (e) calculating interstitial fluid volume (IFV) using the equation IFV = ECFV - TVPV.

In a second aspect, the invention provides an apparatus for simultaneously measuring extracellular fluid volume (ECFV) and total vascular plasma volume (TTPV) of an animal with renal failure according to the first aspect of the invention comprising:

    (a) means for providing the injectate to the vascular system of the animal;
    (b) means for measuring $C_1$ and $C_2$ in vivo in the vascular system of the animal;
    (c) means for calculating ECFV and TVPV;
    (d) means for displaying the calculated values of ECFV and TVPV, and optionally
    (e) means for calculating IFV and means for displaying the calculated value of IFV.

In a third aspect, the invention provides an injectate having a volume V containing a first molecule and a second molecule, wherein the first molecule (i) is dextran with a molecular size of 1 kDa to 20 kDa labeled with a first fluorescent dye having a first excitation wavelength and a first emission wavelength, (ii) is non-metabolized and permeable to vessel walls of the vascular system of the animal and (iii) has a first emission fluorescence intensity of $F_1$, and wherein the second molecule (i) is dextran with a molecular size of 70 kDa to 500 kDa labeled with a second fluorescent dye having a second excitation wavelength and a second emission wavelength, (ii) is non-metabolized and impermeable to the vessel walls of the vascular system of the animal and (iii) has a second emission fluorescence intensity of $F_2$, for use in a method for simultaneously measuring extracellular fluid volume (ECFV) and total vascular plasma volume (TVPV) in an animal with renal failure, the method comprising:

    (a) administering the injectate into the vascular system of the animal;
    (b) allowing the first molecule and the second molecule to each reach an equilibrium steady state concentration within the vascular system of the animal;
    (c) exciting the first molecule in vivo in the vascular system of the animal with a first excitation light source having a first excitation wavelength and exciting the second molecule in vivo in the vascular system of the animal with a second excitation light source having a second excitation wavelength;
    (d) measuring the third emission fluorescence intensity $F_3$ from the first molecule in vivo in the vascular system of the animal and measuring the forth emission fluorescence intensity $F_4$ from the second molecule in vivo in the vascular system of the animal; and
    (e) calculating the ECFV using the equation ECFV = $(F_1 * V)/F_3$ and the TVPV using the equation TVPV = $(F_2 * V)/F_4$.

Also disclosed is a method for measuring extracellular fluid volume (ECFV) in an animal with renal failure. The method comprises: (a) administering a sufficient amount ($A_1$) of a first molecule to the vascular system of the animal wherein the first molecule is non-metabolized and permeable to vessel walls of the vascular system; (b) allowing the first molecule to reach a first equilibrium steady state concentration ($C_1$) in the vascular system of the animal; (c) measuring the $C_1$ in the vascular system of the animal; and (d) calculating the ECFV using the equation: ECFV = $A_1/C_1$. The first molecule may be administered by intravenous injection of an injectate containing the first molecule. The intravenous injection can be bolus or continuous infusion. Alternatively, the first molecule may be administered by inhalation.

Disclosed is an embodiment in which the first molecule has a molecular size of from about 1 kDa to about 20 kDa.

Disclosed is another embodiment in which the first molecule is dextran. Disclosed is yet another embodiment in which the first molecule is labeled with a first fluorescent dye and detected and quantified by the fluorescence intensity of the molecule. The first fluorescent dye can be selected from, but not limited to, xanthene dye, CAL flour, Alexa Flour, Oregon green, carbocyanine, fluorescein, fluorescein isothiocyanate (FITC), carboxy fluoresecein, cyanine, rhodamine, tetramethylrhodamine (Tamra), tetrmethyl rhodamine isothiocyanate (TRITC), X rhodamine isothiocyanate (XRITC), Texas red, DiLight and indocyannine green (ICG).

Measurement of the concentration of the first molecular dye in the vascular system can be performed in vitro or in vivo. In the in vitro method, a sample of blood is drawn from the animal after the first molecule has reached a steady state equilibrium concentration in the vascular system of the animal. A plasma or serum supernatant is prepared from the blood sample by a method such as, but not limited to, centrifugation or filtration. The fluorescence intensity of the first molecule is measured in the supernatant. In the in vivo method, the fluorescence intensity of the first molecule is measured directly in vivo within the vascular system of the animal without having to remove a blood sample from the animal. A preferred method for in vivo measurement of the first molecule is to use a first molecule labeled with a first fluorescent dye.

Also disclosed is a method for determining extracellular fluid volume (ECFV) in an animal with renal failure comprising: (a) providing a first injectate having a volume $V_1$ containing a first molecule labeled with a first fluorescent dye having a first excitation wavelength and a first emission wavelength, wherein the first molecule is non-metabolized and permeable to vessel walls of the vascular system and the first injectate has a first emission fluorescence intensity of $F_1$; (b) administering the first injectate to the vascular system of the animal; (c) allowing the first molecule to reach a first equilibrium steady state concentration in the vascular system of the animal; (d) exciting the first molecule with the first excitation wavelength in vivo in the vascular system of the animal; (e) measuring the second emission fluorescence intensity $F_2$ of the first molecule in vivo in the vascular system of the animal; and (f) calculating the ECFV using the equation:

$$ECFV = (F_1 * V_1)/F_2.$$

Also disclosed is a method for determining total vascular plasma volume (TVPV) in an animal comprising: (a) administering a sufficient amount ($A_2$) of a second molecule to the vascular system of the animal, wherein the second molecule is non-metabolized and impermeable to vessel walls of the vascular system; (b) allowing the second molecule to reach a second equilibrium steady state concentration in the plasma within the vascular system of the animal; (c) measuring the second equilibrium steady state concentration ($C_2$) of the second molecule; and calculating the TVPV using the equation:

$$TVPV = A_2/C_2.$$

Disclosed is an embodiment in which the second molecule has a molecular size of from about 70 kDa to about 500 kDa. Disclosed is another embodiment in which the second molecule is a dextran. Disclosed is yet another embodiment in which the second molecule is labeled with a second fluorescent dye and detected by the emission fluorescence intensity of the molecule. The second fluorescent dye can be selected from, but not limited to, xanthene dye, CAL flour, Alexa Flour, Oregon green, carbocyanine, fluorescein, fluorescein isothiocyanate (FITC), carboxy fluoresecein, cyanine, rhodamine, tetramethylrhodamine (Tamra), tetrmethyl rhodamine isothiocyanate (TRITC), X rhodamine isothiocyanate (XRITC), Texas red, DiLight and indocyannine green (ICG).

Measurement of the concentration of the second molecule in the vascular system can be performed in vitro or in vivo. In the in vitro method, a sample of blood is drawn from the animal after the second molecule has reached a steady state equilibrium concentration in the vascular system of the animal. A plasma or serum supernatant is prepared from the blood sample by a method such as, but not limited to, centrifugation or filtration. The concentration of the second molecule is measured in the plasma or serum supernatant. In the in vivo method, the second molecule is measured directly in vivo within the vascular system of the animal without having to remove a blood sample from the animal. A preferred method for in vivo measurement of the second molecule is to use a second molecule labeled with a second fluorescent dye.

Also disclosed is a method for determining total vascular plasma volume (TVPV) in an animal comprising: (a) providing a second injectate having a volume $V_2$ containing a second molecule labeled with a second fluorescent dye having a second excitation wavelength and a second emission wavelength, wherein the second molecule is non-metabolized and impermeable to vessel walls of the vascular system and the second injectate has a third emission fluorescence intensity of $F_3$; (b) administering the second injectate to the vascular system of the animal; (c) allowing the second molecule to reach a second equilibrium steady state concentration in the vascular system of the animal; (d) exciting the second molecule with the second excitation wavelength in vivo in the vascular system of the animal; (e) measuring the forth emission fluorescence intensity $F_4$ of the second molecule in vivo in the vascular system of the animal; and (f) calculating the TVPV using the equation: TVPV = $(F_3 * V_2)/F_4$.

Also disclosed is a method for determining the interstitial fluid volume (IFV) in an animal comprising: (a) determining the extracellular fluid volume (ECFV) of the animal; (b) determining the total vascular plasma volume (TVPV) of the animal; and (c) calculating the IFV of the animal using the equation: IFV = ECFV - TVPV.

Also disclosed is a method for simultaneously measuring extracellular fluid volume (ECFV) and total vascular plasma volume (TVPV) in an animal with renal failure comprising: (a) providing an injectate containing a known amount $A_1$ of a first molecule and a known amount $A_2$ of a second molecule, wherein the first molecule is non-metabolized and permeable to vessel walls of the vascular system of the animal and the second molecule is non-metabolized and impermeable to vessel walls of the vascular system of the animal; (b) administering the injectate into the vascular system of the animal; (c) allowing the first molecule to reach a first equilibrium steady state concentration $C_1$ and the second molecule to reach a second equilibrium steady state concentration $C_2$; (d) measuring $C_1$ and $C_2$ in the vascular system of the animal; and (e) calculating ECFV using the equation ECFV = $A_1/C_1$ and TVPV using the equation TVPV = $A_2/C_2$.

In the first aspect of the invention, measurement of the concentration of the first molecule and the second molecule in the vascular system can be performed in vitro or in vivo. In the in vitro method, a sample of blood is drawn from the animal after the first molecule and the second molecule have each reached a steady state equilibrium concentration in the vascular system of the animal. A plasma or serum supernatant is prepared from the blood sample by a method such as, but not limited to, centrifugation or filtration. The concentration of the first molecule and the second molecule is measured in the supernatant. In the in vivo method, the first molecule and the second molecule are measured directly in vivo within the vascular system of the animal without having to remove a blood sample from the animal. A preferred method for in vivo measurement of the first molecule and the second molecule is to use a first molecule labeled with a first fluorescent dye and a second molecule labeled with a second fluorescent dye that is different from the first dye.

The method may further comprise an additional step of calculating the interstitial fluid volume (IFV) using the equation: IFV = ECFV - TVPV.

An apparatus for determining the ECVF and TVPV using these methods may comprise: (a) means for providing the injectate to the vascular system of the animal; (b) means for measuring $C_1$ and $C_2$ in vivo in the vascular system of the animal; (c) means for calculating ECFV and TVPV; and (d) means for displaying the calculated values of ECFV and TVPV. Optionally, the apparatus may further comprise means for calculating IFV and displaying the calculated value of IFV. The apparatus may be a stand alone unit or incorporated into a hemodialysis device.

Also disclosed is a method for simultaneously measuring extracellular fluid volume (ECFV) and total vascular plasma volume (TVPV) in an animal with renal failure comprising: (a) providing an injectate having a volume V containing a first molecule and a second molecule, wherein the first molecule (i) is labeled with a first fluorescent dye having a first excitation wavelength and a first emission wavelength, (ii) is non-metabolized and permeable to vessel walls of the vascular system of the animal and (iii) has a first emission fluorescence intensity of $F_1$, and wherein the second molecule (i) is labeled with a second fluorescent dye having a second excitation wavelength and a second emission wavelength, (ii) is non-metabolized and impermeable to vessel walls of the vascular system of the animal and (iii) has a second emission fluorescence intensity of $F_2$; (b) administering the injectate into the vascular system of the animal; (c) allowing the first molecule and the second molecule to each reach equilibrium steady state concentrations within the vascular system of the animal; (d) exciting the first molecule in vivo in the vascular system of the animal with a first excitation light source having a first excitation wavelength and exciting the second molecule in vivo in the vascular system of the animal with a second excitation light source having a second excitation wavelength; (e) measuring the third emission fluorescence intensity $F_3$ from the first molecule in vivo in the vascular system of the animal and measuring the forth emission fluorescence intensity $F_4$ from the second molecule in vivo in the vascular system of the animal; and (f) calculating the ECFV using the equation ECFV = $(F_1 * V)/F_3$ and the TVPV using the equation TVPV = $(F_2 * V)/F_4$.

The method may further comprise an additional step of calculating the interstitial fluid volume (IFV) using the equation: IFV = ECFV - TVPV.

Other features and advantages of the invention will be apparent from the following specification taken in conjunction with the following drawings.

BRIEF DESCRIPTION OF THE DRAWINGS:

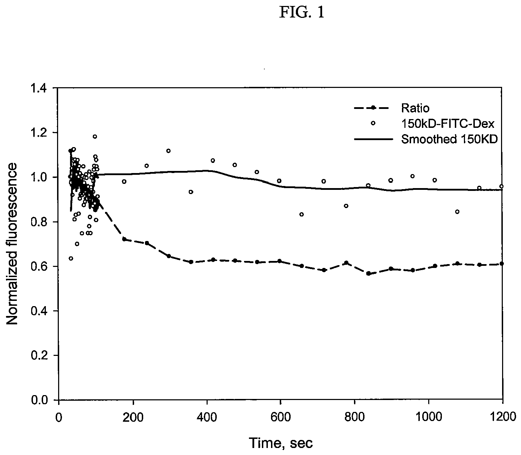

FIG. 1 shows a decay curve ($\bigcirc$) of the fluorescence from the larger fluorescent marker 150-kDa FITC-dextran which was administered to a bilaterally anephric rat as described in Example 1. Also shown is the smoothed fluorescence curve (---) of the fluorescence from the 150-kDa FITC-dextran as well as a decay curve of the ratio of the fluorescence from the 3-kDa Texas Red-dextran to that of the 150-kDa FITC dextran (---●---).

DETAILED DESCRIPTION

While this invention is susceptible of embodiments in many different forms, there is shown in the drawings and will herein be described in detail preferred embodiments of the invention with the understanding that the present disclosure is to be considered as an exemplification of the principles of the invention and is not intended to limit the broad aspect of the invention to the embodiments illustrated.

The present invention is related generally to measurement of body fluid volumes in an animal subject. The body fluid volumes of interest include extracellular fluid volume (ECFV), total vascular plasma volume (TVPV) and interstitial fluid volume (IFV). The methods are especially beneficial for subjects suffering from renal failure and particularly those undergoing renal dialysis. The animal subject may be a mammalian subject, and the mammalian subject may be a human. The renal failure may be acute or chronic. Acute renal failure may be due to acute renal injury, and chronic renal failure may be due to late stage renal disease (ESRD). Renal dialysis can be hemodialysis or peritoneal dialysis if the abdominal cavity is dry. The renal failure may also be temporary or permanent.

In brief, ECFV can be measured by administering a first molecule which is non-metabolized and permeable to vessel walls of the vascular system wherein the first molecule is distributed within the total vascular spaces as well as the interstitial spaces. TVPV can be measured by administering a second molecule which is non-metabolized and impermeable to vessel walls of the vascular system wherein the second molecule is distributed within only the vascular spaces. IFV can then be calculated using the equation IFV = ECFV - TVPV.

What is meant by total vascular plasma volume (TVPV) as used in the present application is the amount of plasma volume contained within the entire vascular space including arterial, venous and capillary spaces. The TVPV does not include the volume contributed by the blood cells, such as the red blood cells. TVPV may also be referred to as the Plasma Volume (PV). What is meant by interstitial fluid volume (IFV) as used in the present application is the amount of volume extra vascular and surrounding cells as well as collections of fluid such as ascites or pleural fluid. IFV is a good indicator for capillary leakage. Expanded IFV is indicative that fluid is leaking from the vascular system and accumulating into the interstitial space which results in edema. The extracellular fluid volume (ECFV) as used in the present application is the sum of the TVPV and IFV. The relationship between these volumes can, therefore, be represented by the following equation:

$$ECFV = TVPV + IFV \qquad (1)$$

Total blood volume (TBV) can be estimated from the TVPV by adding TVPV and the volume contributed by the blood cells, which can be determined from the Hematocrit (Hct) or from the Packed Cell Volume (PCV).

Disclosed herein is a method for measuring extracellular fluid volume (ECFV) in an animal with renal failure. The method comprises: (a) administering a sufficient amount ($A_1$) of a first molecule to the vascular system of the animal wherein the first molecule is non-metabolized and permeable to vessel walls of the vascular system; (b) allowing the first molecule to reach a first equilibrium steady state concentration ($C_1$) in the vascular system of the animal; (c) measuring the $C_1$ in the vascular system of the animal; and (d) calculating the ECFV using the equation: ECFV = $A_1/C_1$.

Also disclosed herein are methods for determining total vascular plasma volume (TVPV) in an animal comprising: (a) administering a sufficient amount ($A_2$) of a second molecule to the vascular system of the animal, wherein the second molecule is non-metabolized and impermeable to vessel walls of the vascular system; (b) allowing the second molecule to reach a second equilibrium steady state concentration in the plasma within the vascular system of the animal; (c) measuring the second equilibrium steady state concentration ($C_2$) of the second molecule; and calculating the TVPV using the equation:

$$TVPV = A_2/C_2.$$

Once the ECFV and the TVPV are determined, interstitial fluid volume (IFV) can be calculated using the equation:

$$IFV = ECFV - TVPV \qquad (2)$$

What is meant by a sufficient amount of the first molecule or the second molecule is that the molecule is above the detection limit using an appropriate analytical technique after the molecule has reached equilibrium following distribution. The appropriate analytical method depends on the properties and characteristics of the molecule. Examples of commonly used analytical method include but are not limited to absorption spectroscopy, fluorescence, adsorption, and radioactive activity of the molecule.

The time for the first molecule or the second molecule to reach its respectively steady state equilibrium concentration depends on the molecule and the animal species. Such time for reaching equilibrium can easily be determined by dosing the animal with the molecule and monitoring the molecule in the vascular system of the animal over time. Initially, the concentration of the molecule rises in the vascular system, which represents a mixing phase of the molecule in the vascular system. Eventually, the concentration of the molecule reaches an equilibrium steady state in the vascular system when the concentration plateaus. The beginning of the plateau of the concentration of the molecule marks the end of the mixing phase. An example of such a method is described in Example 1 below. This equilibrium time is relatively constant for a specific molecule and a specific animal species so that once such time is determined for the molecule and the animal species, the value can be used for the same molecule and the same animal species without having to determine the value again. In human beings, the equilibrium time is about 10 to 15 minutes for most molecules. However, in certain disease states, such as congestive heart failure, it may take longer time to reach equilibrium.

The first molecule or the second molecule may be administered by a suitable method such as intravenous injection of an injectate containing the first molecule and/or the second molecule. The intravenous injection can be bolus or continuous infusion over a period of time.

What is meant by "non-metabolized" is that the molecule is not significantly metabolized by the animal during the time in which the measurements are performed. What is meant by "permeable to vessel walls" refers to that the molecule can cross the vessel walls. This movement of the molecule can be a passive method without requiring energy, e.g. diffusion, or an active method requiring energy, e.g. active transport. Similarly, "impermeable to vessel walls" refers to that the molecule cannot cross the vessel walls either through a passive process or an active process.

According to the present invention, the first molecule has a molecular size of from 1 kDa to 20 kDa, and the second molecule has a molecule size of from 70 kDa to 500 kDa. The first and the second molecule are dextrans. In some methods disclosed herein, the first molecule or the second molecule is a fluorescent molecule. In the first aspect of the invention the first molecule is labelled with a first fluorescent dye and the second molecule is labelled with a second fluorescent dye. In the third aspect of the invention the first molecule is a dextran labeled with a first fluorescent dye having a first excitation wavelength and a first emission wavelength , and the second molecule is a dextran labeled with a second fluorescent dye having a second excitation wavelength and a second emission wavelength. The first or second fluorescent dye can be selected from, but not limited to, xanthene dye, CAL flour, Alexa Flour, Oregon green, carbocyanine, fluorescein, fluorescein isothiocyanate (FITC), carboxy fluoresecin, cyanine, rhodamine, tetramethylrhodamine (Tamra), tetrmethyl rhodamine isothiocyanate (TRITC), X rhodamine isothiocyanate (XRITC), Texas red, DiLight and indocyannine green (ICG). The first or second fluorescent dye can be the same or different. In some methods disclosed herein, the first molecule may be administered separately from the second molecule, or they may be administered simultaneously.

Measurement of the concentration of the first molecule or the second molecule in the vascular system can be performed in vitro or in vivo. In the in vitro method, a sample of blood is drawn from the animal after the first molecule or the second molecule has reached a steady state equilibrium concentration in the vascular system of the animal. A plasma or serum supernatant of the blood is prepared from the blood sample by a method which removes the blood cells from the blood, such as, but not limited to, centrifugation or filtration. These separation methods are well known to those skilled in the art and are routinely practiced in the laboratory. The supernatant represents the plasma of the blood. The concentration of the first molecule or the second molecule is measured in the supernatant by an appropriate detection method such as absorption spectroscopy or fluorescence. In the in vivo method, the first or the second molecule is measured directly in vivo within the vascular system of the animal without having to remove a blood sample from the animal. A preferred method for in vivo measurement of a molecule is to use a molecule labeled with a fluorescent dye. An example of an in vivo measurement of a fluorescent molecule in the vascular system of the animal has been disclosed in a pending United States Patent 12/425,827.

The method is applicable to measuring one or more fluorescent molecules simultaneously in vivo.

In methods for measuring ECVF in an animal with renal failure in which the first molecule is a fluorescent molecule, the method may comprise: (a) providing a first injectate containing the first molecule having a first excitation wavelength and a first emission wavelength, the first injectate having a volume of $V_1$ wherein the first molecule is non-metabolized and permeable to vessel walls of the vascular system of the animal; (b) measuring a first emission fluorescence intensity $F_1$ of the first molecule in the first injectate; (c) administering the first injectate into the vascular system of the animal; (d) allowing the first molecule to reach a steady state equilibrium concentration; (e) measuring a second emission fluorescence intensity $F_2$ of the first molecule in the vascular system; and (f) calculating the ECVF using the equation" ECVF = $(F_1 * V_1)/F_2$. $F_2$ may be measured in vitro or in vivo.

Where $F_2$ is measured in vivo, the method comprises: (a) providing a first injectate having a volume $V_1$ containing

a first molecule labeled with a first fluorescent dye having a first excitation wavelength and a first emission wavelength, wherein the first molecule is non-metabolized and permeable to vessel walls of the vascular system and the first injectate has a first emission fluorescence intensity of $F_1$; (b) administering the first injectate to the vascular system of the animal; (c) allowing the first molecule to reach a first equilibrium steady state concentration in the vascular system of the animal; (d) exciting the first molecule with the first excitation wavelength in vivo in the vascular system of the animal; (e) measuring the second emission fluorescence intensity $F_2$ of the first molecule in vivo in the vascular system of the animal; and (f) calculating the ECFV using the equation:

$$ECFV = (F_1 * V_1)/F_2.$$

Similarly, in methods for measuring TVPV in an animal in which the second molecule is a fluorescent molecule, the method may comprise: (a) providing a second injectate containing the second molecule having a second excitation wavelength and a second emission wavelength, the second injectate having a volume of $V_2$ wherein the second molecule is non-metabolized and impermeable to vessel walls of the vascular system of the animal; (b) measuring a third emission fluorescence intensity $F_3$ of the second molecule in the second injectate; (c) administering the second injectate into the vascular system of the animal; (d) allowing the second molecule to reach a steady state equilibrium concentration; (e) measuring a forth emission fluorescence intensity $F_4$ of the second molecule in the vascular system; and (f) calculating the ECVF using the equation" TVPV = $(F_3 * V_2)/F_4$. $F_4$ may be measured in vitro or by in vivo.

Where $F_4$ is measured in vivo, the method comprises: (a) providing a second injectate having a volume $V_2$ containing a second molecule labeled with a second fluorescent dye having a second excitation wavelength and a second emission wavelength, wherein the second molecule is non-metabolized and impermeable to vessel walls of the vascular system and the second injectate has a third emission fluorescence intensity of $F_3$; (b) administering the second injectate to the vascular system of the animal; (c) allowing the second molecule to reach a second equilibrium steady state concentration in the vascular system of the animal; (d) exciting the second molecule with the second excitation wavelength in vivo in the vascular system of the animal; (e) measuring the forth emission fluorescence intensity $F_4$ of the second molecule in vivo in the vascular system of the animal; and (f) calculating the TVPV using the equation: TVPV = $(F_3 * V_2)/F_4$.

Disclosed herein are methods for simultaneously measuring extracellular fluid volume (ECFV) and total vascular volume (TVPV) in an animal with renal failure comprising: (a) providing an injectate containing a known amount $A_1$ of a first molecule and a known amount $A_2$ of a second molecule, wherein the first molecule is non-metabolized and permeable to vessel walls of the vascular system of the animal and the second molecule is non-metabolized and impermeable to vessel walls of the vascular system of the animal; (b) administering the injectate into the vascular system of the animal; (c) allowing the first molecule to reach a first equilibrium steady state concentration $C_1$ and the second molecule to reach a second equilibrium steady state concentration $C_2$; (d) measuring $C_1$ and $C_2$ in the vascular system of the animal; and (e) calculating ECFV using the equation ECFV = $A_1/C_1$ and TVPV using the equation TVPV = $A_2/C_2$.

Measurement of the concentration of the first molecular and the second in the vascular system can be performed in vitro or in vivo. In the in vitro method, a sample of blood is drawn from the animal after the first molecule and the second molecule have each reached a steady state equilibrium concentration in the vascular system of the animal. A plasma or serum supernatant is prepared from the blood sample by a method such as, but not limited to, centrifugation or filtration. The concentration of the first molecule and the second molecule is measured in the supernatant. In the in vivo method, the first molecule and the second molecule are measured directly in vivo within the vascular system of the animal without having to remove a blood sample from the animal. A preferred method for in vivo measurement of the first molecule and the second molecule is to use a first molecule labeled with a first fluorescent dye and a second molecule labeled with second fluorescent dye.

The method may further comprise an additional step of calculating the interstitial fluid volume (IFV) using the equation: IFV = ECFV - TVPV.

An apparatus for determining the ECFV and TVPV using these methods may comprise: (a) means for providing the injectate to the vascular system of the animal; (b) means for measuring $C_1$ and $C_2$ in vivo in the vascular system of the animal; (c) means for calculating ECFV and TVPV; and (d) means for displaying the calculated values of ECFV and TVPV. Optionally, the apparatus may further comprise means for calculating IFV and displaying the calculated value of IFV. The apparatus may be a stand alone unit or incorporated into a hemodialysis device.

Disclosed herein are methods for simultaneously measuring extracellular fluid volume (ECFV) and total vascular plasma volume (TVPV) in an animal with renal failure comprising: (a) providing an injectate having a volume V containing a first molecule and a second molecule, wherein the first molecule (i) is labeled with a first fluorescent dye having a first excitation wavelength and a first emission wavelength, (ii) is non-metabolized and permeable to vessel walls of the vascular system of the animal and (iii) has a first emission fluorescence intensity of $F_1$, and wherein the second molecule (i) is labeled with a second fluorescent dye having a second excitation wavelength and a second emission wavelength, (ii) is non-metabolized and impermeable to the vessel walls of the vascular system of the animal and (iii) has a second emission fluorescence intensity of $F_2$; (b) administering the injectate into the vascular system of the animal; (c) allowing the first molecule and the second molecule to each reach an equilibrium steady state concentration within the vascular system of the animal; (d) exciting the first molecule in vivo in the vascular system of the animal with a first excitation light source having a first excitation wavelength and exciting the second molecule in vivo in the vascular system of the animal with a second excitation light source having a second excitation wavelength; (e) measuring the third emission fluorescence intensity $F_3$ from the first molecule in vivo in the vascular system of the animal and measuring the forth emission fluorescence intensity $F_4$ from the second molecule in vivo in the vascular system of the animal; and (f) calculating the ECFV using the equation ECFV = $(F_1 * V)/F_3$ and the TVPV using the equation TVPV = $(F_2 * V)/F_4$.

The method may further comprise an additional step of calculating the interstitial fluid volume (IFV) using the equation: IFV = ECFV - TVPV.

## EXAMPLES

Example 1: Measurement of TVPV and ECFV in bilaterally anephric rats

The example shown here was a test conducted on a bilaterally anephric rat, which was infused with a mixture of 3 kDa Texas Red-dextran and 150 kDa FITC-dextran. The dynamic plasma fluorescence intensity was obtained by *in vivo* two-photon liver imaging of vascular plasma. Only the vascular plasma containing regions in each image were included for calculation. The decay curve of the fluorescence intensity of the 150-kDa FITC-dextran as well as the decay curve of the ratio of the fluorescence intensity of the Texas Red-dextran to that of the FITC-dextran after the infusion is shown in FIG. 1. Using the ratio rather than the 3 kDa Texas Red dextran or the 150 kDa FITC-dextran signal directly helped reduce the signal fluctuation caused by focus movement during imaging since the same fluctuation showed up in both channels.

To test if the volumes determined by this method agree with expected values we injected a mixture of 3KDa TexasRed-dextran and 150kDa FITC-dextran to two bilaterally anephric rats. Blood was drawn from the animals 15 minutes after the infusion. According to the FIG. 1, this should be more than enough time for the dextrans to become equilibrated between the vascular and the interstitial spaces. The blood plasma was then separated by centrifuge. Fluorescence was measured using a spectrophotometer. TVPV and ECFV from each rat were determined using eqs. 4 and 5, respectively. The measured volumes along with estimated plasma volumes by body weight are shown in the following table.

Table 1: Measured and Estimated Plasma Volumes in Anephric Rats

|       | Measured TVPV (ml) | Estimated TVPV (ml) | Measured ECFV (ml) | Calculated IFV |
|-------|--------------------|---------------------|--------------------|----------------|
| Rat 1 | 8.30               | 7.95                | 22.94              | 14.64          |
| Rat 2 | 6.32               | 6.77                | 18.12              | 11.80          |

Estimated TVPV values were obtained from a method described by Altman P. L. ("Blood and Other Body Fluids" Fed of Am Societies for Experimental Biology (1961) Washington, D.C.) and Yu W. et al. ("Rapid Determinations of Renal Filtration Function using an Optical Ratiometric Image Approach" Am J Physiology - Renal Physiology (2007) 292(6): F1873-80).

IFV can be calculated from the measured TVPV and ECFV using the equation IFV = ECFV - TVPV.

Example 2: Anticipated minimally invasive method for measuring fluid volumes in a patent with renal failure

We anticipate developing a minimally invasive method for measuring TVPV, ECFV and IFV in a patient with renal failure using a small dextran (molecule size of about 1 kDa to about 20 kDa) labeled with a first fluorescent dye to distribute to the vascular and interstitial spaces and a large dextran (molecule size of about 70 kDa to about 500 kDa) labeled with a second fluorescent dye to distribute only to the vascular space of the animal. The molecules can be simultaneously detected in vivo using a dual channel fluorescence detection device and a proprietary fiber optic catheter. The fluorescence device and the fiber optic catheter have both been disclosed in a pending United States Patent Application No. 12/425,827 and, more specifically, for this specific subject matter disclosed at Paragraphs [0077] to [0093] and FIGS. 1 and 91-14 for the detector and Paragraphs [0108] to [0112] and FIGS. 1, 16, and 17 for the fiber optic catheter.

The method comprises: (1) inserting the proprietary fiber optic catheter into a peripheral vein in the patient's upper extremity; (2) connecting the fiber optic catheter to the fluorescence device; (3) attaching a syringe containing 5 to 10 ml of an injectate containing the small and large fluorescent dextrans to the catheter; (4) injecting 1 ml of the injectate into the calibration chamber of the catheter, and backfilling with patient's blood; (5) calibrating the fluorescence detection device; (6) advancing the fiber optic line through the catheter and into the catheter; (7) allowing enough time (approximately 10 to 15 minutes) for the molecules to equilibrate in the patient; (8) detecting the fluorescence intensities of the small and large dextrans with the fluorescence device; (9) calculating the fluid volumes using a preprogrammed algorithm; and (10) displaying the values of the fluid volumes on a screen.

Some of the key advantages of this method are that it is fast (only takes about 15 minutes), accurate and inexpensive. More importantly, the fluid volumes can be determined using data from a single time point.

While the specific embodiments have been illustrated and described, numerous modifications come to mind and the scope of protection is only limited by the scope of the accompanying Claims.

## REFERENCES

1. Agarwal R. et al. "Diagnostic Utility of Blood Volume Monitoring in Hemodialysis Patients" Am J of Kidney Diseases (2008) 51: 242-254.

2. Rodriguez H. J. et al. "Assessment of Dry Weight by Monitoring Changes in Blood Volume During Hemodialysis using Crit-Line" Kidney International (2005) 68, 854-861.

3. Kraemer M. et al. "Detection Limit of Methods to Assess Fluid Status Changes in Dialysis Patients" Kidney International (2006) 69: 1609-1620.

4. Dasselaar J. J. et al. "Measurement of Relative Blood Volume Changes During Haemodialysis: Merits and Limitations" Nephrol Dial Transplant (2005) 20: 2043-2049.

5. Mitra, S. et al. "Serial Determinations of Absolute Plasma Volume with Indocyanine Green During Hemodialyais," J Am Soc of Nephrology. (2003) 14(9): 2345-51.

6. Zhu et al. "Segment-Specific Resistivity Improves Body Fluid Volume Estimates from Bioimpedence Spectroscopy in Hemodialysis Patients" J Appl Physio (2006) 100: 717-724.

7. De Lorenzo A. et al. "Predicting Body Cell Mass With Bioimpedance by Using Theoretical Methods: a Technological Review" J Appl Physiology (1997) 82: 1542-1558.

8. Kuhlmann, M. K. et al. "Bioimpedence, Dry Weight and Blood Pressure Control: New Methods and Consequences" Current Opinion in Nephrology and Hypertension (2005) 14: 543-549.

9. Altman P. L. "Blood and Other Body Fluids" Fed of Am Societies for Experimental Biology (1961) Washington, D.C.

10. Yu W. et al. "Rapid Determinations of Renal Filtration Function using an Optical Ratiometric Image Approach" Am J Physiology - Renal Physiology (2007) 292(6): F1873-80.

## Claims

1. An injectate containing a known amount $A_1$ of a first molecule and a known amount $A_2$ of a second molecule, wherein the first molecule is dextran with a molecular size of 1 kDa to 20 kDa labeled with a first fluorescent dye and is non-metabolized and permeable to vessel walls of the vascular system of the animal, and the second molecule is dextran with a molecular size of 70 kDa to 500 kDa labeled with a second fluorescent dye molecule and is non-metabolized and impermeable to vessel walls of the vascular system of the animal, for use in a method for simultaneously measuring extracellular fluid volume (ECFV) and total vascular plasma volume (TVPV) in an animal with renal failure, the method comprising:

   (a) administering the injectate into the vascular system of the animal;
   (b) allowing the first molecule to reach a first equilibrium steady state concentration $C_1$ and the second molecule to reach a second equilibrium steady state concentration $C_2$;

(c) measuring $C_1$ and $C_2$ in the vascular system of the animal;

(d) calculating ECFV using the equation ECFV = $A_1/C_1$ and TVPV using the equation TVPV = $A_2/C_2$, and optionally

(e) calculating interstitial fluid volume (IFV) using the equation IFV = ECFV - TVPV.

2. The injectate for use of claim 1 wherein the step of measuring $C_1$ and the step of measuring $C_2$, includes:

   (a) withdrawing a sample of blood from the vascular system of the animal;
   (b) obtaining a plasma supernatant from the blood sample; and
   (c) measuring $C_1$ and measuring $C_2$ in the supernatant of the sample.

3. The injectate for use of claim 1 or claim 2 wherein the step of measuring $C_1$ and the step of measuring $C_2$ is performed in vivo.

4. An apparatus for simultaneously measuring extracellular fluid volume (ECFV) and total vascular plasma volume (TVPV) of an animal with renal failure in claim 1 comprising:

   (a) means for providing the injectate to the vascular system of the animal;
   (b) means for measuring $C_1$ and $C_2$ in vivo in the vascular system of the animal;
   (c) means for calculating ECFV and TVPV;
   (d) means for displaying the calculated values of ECFV and TVPV, and optionally
   (e) means for calculating IFV and means for displaying the calculated value of IFV.

5. The apparatus of claim 4 wherein the apparatus is incorporated into a hemodialysis device.

6. An injectate having a volume V containing a first molecule and a second molecule, wherein the first molecule (i) is dextran with a molecular size of 1 kDa to 20 kDa labeled with a first fluorescent dye having a first excitation wavelength and a first emission wavelength, (ii) is non-metabolized and permeable to vessel walls of the vascular system of the animal and (iii) has a first emission fluorescence intensity of $F_1$, and wherein the second molecule (i) is dextran with a molecular size of 70 kDa to 500 kDa labeled with a second fluorescent dye having a second excitation wavelength and a second emission wavelength, (ii) is non-metabolized and impermeable to the vessel walls of the vascular system of the animal and (iii) has a second emission fluorescence intensity of $F_2$, for use in a method for simultaneously measuring extracellular fluid volume (ECFV) and total vascular plasma volume (TVPV) in an animal with renal failure, the method comprising:

   (a) administering the injectate into the vascular system of the animal;
   (b) allowing the first molecule and the second molecule to each reach an equilibrium steady state concentration within the vascular system of the animal;
   (c) exciting the first molecule in vivo in the vascular system of the animal with a first excitation light source having a first excitation wavelength and exciting the second molecule in vivo in the vascular system of the animal with a second excitation light source having a second excitation wavelength;
   (d) measuring the third emission fluorescence intensity $F_3$ from the first molecule in vivo in the vascular system of the animal and measuring the forth emission fluorescence intensity $F_4$ from the second molecule in vivo in the vascular system of the animal; and
   (e) calculating the ECFV using the equation ECFV = $(F_1 * V)/F_3$ and the TVPV using the equation TVPV = $(F_2 * V)/F_4$.

7. The injectate for use of claim 6, wherein the method comprises an additional step of calculating interstitial fluid volume (IFV) using the equation IFV = ECFV - TVPV.

8. The injectate for use of any of claims 1-3 or 6-7, wherein the administration is by intravenous injection, optionally wherein the injection is a bolus injection or an infusion.

9. The injectate for use of any of claims 1-3 or 6-8, wherein the first fluorescent dye and the second fluorescent dye are selected from the group consisting of: xanthene dye, CAL flour, Alexa Flour, Oregon green, carbocyanine, fluorescein, fluorescein isothiocyanate (FITC), carboxy fluorescein, cyanine, rhodamine, tetramethylrhodamine (Tamra), tetramethyl rhodamine isothiocyanate (TRITC), X rhodamine isothiocyanate (XRITC), Texas red, DiLight and indocyanine green (ICG).

**Patentansprüche**

1. Injektat mit einer bekannten Menge $A_1$ eines ersten Moleküls und einer bekannten Menge $A_2$ eines zweiten Moleküls, wobei das erste Molekül Dextran mit einer Molekülgröße von 1 kDa bis 20 kDa ist, mit einem ersten fluoreszierenden Farbstoff markiert, nichtmetabolisiert und für die Gefäßwände des Gefäßsystems des Tieres permeabel, und das zweite Molekül Dextran mit einer Molekülgröße von 70 kDa bis 500 kDa ist, mit einem zweiten fluoreszierenden Farbstoff markiert, nichtmetabolisiert und für die Gefäßwände des Gefäßsystems des Tieres impermeabel, zur Verwendung zum gleichzeitigen Messen von extrazellulärem Flüssigkeitsvolumen (ECFV) und einem gesamten vaskulären Plasmavolumen (TVPV) in einem Tier mit Nierenversagen, wobei das Verfahren Folgendes umfasst:

   (a) Verabreichen des Injektats in das Gefäßsystem des Tieres;
   (b) Zulassen, dass das erste Molekül eine erste Gleichgewichtskonzentration $C_1$ erreicht und dass das zweite Molekül eine zweite Gleichgewichtskonzentration $C_2$ erreicht;
   (c) Messen von $C_1$ und $C_2$ im Gefäßsystem des Tieres;
   (d) Berechnen von ECFV unter Einsatz der Gleichung ECFV = $A_1/C_1$ und von TVPV unter Einsatz der Gleichung TVPV = $A_2/C_2$ und optional
   (e) Berechnen eines interstitiellen Flüssigkeitsvolumens (IFV) unter Einsatz der Gleichung IFV = ECFV - TVPV.

2. Injektat zur Verwendung nach Anspruch 1, wobei der Schritt des Messens von $C_1$ und der Schritt des Messens von $C_2$ Folgendes enthalten:

   (a) Entnehmen einer Probe von Blut aus dem Gefäßsystem des Tieres;
   (b) Beschaffen eines Plasmaüberstands aus der Blutprobe; und
   (c) Messen von $C_1$ und Messen von $C_2$ im Überstand der Probe.

3. Injektat zur Verwendung nach Anspruch 1 oder 2, wobei der Schritt des Messens von $C_1$ und der Schritt des Messens von $C_2$ in vivo durchgeführt werden.

4. Vorrichtung zum gleichzeitigen Messen eines extrazellulären Flüssigkeitsvolumens (ECFV) und eines gesamten vaskulären Plasmavolumens (TVPV) eines Tieres mit Nierenversagen in Anspruch 1, Folgendes umfassend:

   (a) Mittel zum Verabreichen des Injektats in das Gefäßsystem des Tieres;
   (b) Mittel zum Messen von $C_1$ und $C_2$ in vivo im Gefäßsystem des Tieres;
   (c) Mittel zum Berechnen von ECFV und TVPV;
   (d) Mittel zum Darstellen der berechneten Werte von ECFV und TVPV und optional
   (e) Mittel zum Berechnen von IFV und Mittel zum Darstellen des berechneten Werts für IFV.

5. Vorrichtung nach Anspruch 4, wobei die Vorrichtung in eine Hämodialysevorrichtung eingebunden ist.

6. Injektat mit einem Volumen V mit einem ersten Molekül und einem zweiten Molekül, wobei das erste Molekül (i) Dextran mit einer Molekülgröße von 1 kDa bis 20 kDa ist, mit einem ersten fluoreszierenden Farbstoff mit einer ersten Anregungswellenlänge und einer ersten Emissionswellenlänge markiert, (ii) nichtmetabolisiert und für die Gefäßwände des Gefäßsystems des Tieres permeabel ist und (iii) eine erste Emissionsfluoreszenzintensität $F_1$ aufweist und wobei das zweite Molekül (i) Dextran mit einer Molekülgröße von 70 kDa bis 500 kDa ist, mit einem zweiten fluoreszierenden Farbstoff mit einer zweiten Anregungswellenlänge und einer zweiten Emissionswellenlänge markiert, (ii) nichtmetabolisiert und für die Gefäßwände des Gefäßsystems des Tieres impermeabel ist und (iii) eine zweite Emissionsfluoreszenzintensität $F_2$ aufweist, zur Verwendung zum gleichzeitigen Messen von extrazellulärem Flüssigkeitsvolumen (ECFV) und einem gesamten vaskulären Plasmavolumen (TVPV) in einem Tier mit Nierenversagen, wobei das Verfahren Folgendes umfasst:

   (a) Verabreichen des Injektats in das Gefäßsystem des Tieres;
   (b) Zulassen, dass das erste Molekül und das zweite Molekül jeweils eine Gleichgewichtskonzentration in dem Gefäßsystem des Tieres erreichen;
   (c) Anregen des ersten Moleküls in vivo in dem Gefäßsystem des Tieres mit einer ersten Anregungslichtquelle mit einer ersten Anregungswellenlänge und Anregen des zweiten Moleküls in vivo in dem Gefäßsystem des Tieres mit einer zweiten Anregungslichtquelle mit einer zweiten Anregungswellenlänge;
   (d) Messen der dritten Emissionsfluoreszenzintensität $F_3$ von dem ersten Molekül in vivo in dem Gefäßsystem des Tieres und Messen der vierten Emissionsfluoreszenzintensität $F_4$ von dem zweiten Molekül in vivo in dem Gefäßsystem des Tieres; und

(e) Berechnen des ECFV unter Einsatz der Gleichung ECFV = $(F_1 * V)/F_3$ und des TVPV unter Einsatz der Gleichung TVPV = $(F_2 * V)/F_4$.

**7.** Injektat zur Verwendung nach Anspruch 6, wobei das Verfahren einen zusätzlichen Schritt des Berechnens von interstitiellem Flüssigkeitsvolumen (IFV) unter Einsatz der Gleichung IFV = ECFV - TVPV umfasst.

**8.** Injektat zur Verwendung nach einem der Ansprüche 1-3 oder 6-7, wobei die Verabreichung durch intravenöse Injektion stattfindet, optional wobei die Injektion eine Bolusinjektion oder eine Infusion ist.

**9.** Injektat zur Verwendung nach einem der Ansprüche 1-3 oder 6-8, wobei der erste fluoreszierende Farbstoff und der zweite fluoreszierende Farbstoff ausgewählt sind aus der Gruppe bestehend aus: Xanthenfarbstoff, CAL-Mehl, Alexa-Mehl, Oregon-Grün, Carbocyanin, Fluorescein, Fluoresceinisothiocyanat (FITC), Carboxyfluorescein, Cyanin, Rhodamin, Tetramethylrhodamin (Tamra), Tetramethylrhodaminisothiocyanat (TRITC), X-Rhodaminisothiocyanat (XRITC), Texas-Rot, DiLight und Indocyanin-Grün (ICG).


**Revendications**

**1.** Produit à injecter contenant une quantité connue $A_1$ d'une première molécule et une quantité connue $A_2$ d'une deuxième molécule, la première molécule étant du dextrane ayant une taille moléculaire de 1 kDa à 20 kDa marqué avec un premier colorant fluorescent et étant non métabolisée et perméable aux parois de vaisseaux du système vasculaire de l'animal, et la deuxième molécule étant du dextrane ayant une taille moléculaire de 70 kDa à 500 kDa marqué avec une deuxième molécule de colorant fluorescent et étant non métabolisée et imperméable aux parois de vaisseaux du système vasculaire de l'animal, pour une utilisation dans un procédé pour mesurer simultanément un volume de fluide extracellulaire (ECFV) et un volume de plasma vasculaire total (TVPV) dans un animal présentant une insuffisance rénale, le procédé comprenant les étapes consistant à :

(a) administrer le produit à injecter dans le système vasculaire de l'animal ;
(b) laisser la première molécule atteindre une première concentration d'état stable d'équilibre $C_1$ et la deuxième molécule atteindre une deuxième concentration d'état stable d'équilibre $C_2$ ;
(c) mesurer $C_1$ et $C_2$ dans le système vasculaire de l'animal ;
(d) calculer l'ECFV en utilisant l'équation ECFV = $A_1/C_1$ et le TVPV en utilisant l'équation TVPV = $A_2/C_2$, et éventuellement
(e) calculer le volume de fluide interstitiel (IFV) en utilisant l'équation IFV = ECFV-TVPV.

**2.** Produit à injecter pour une utilisation de la revendication 1 dans lequel l'étape consistant à mesurer $C_1$ et l'étape consistant à mesurer $C_2$ incluent les étapes consistant à :

(a) prélever un échantillon de sang à partir du système vasculaire de l'animal ;
(b) obtenir un surnageant de plasma à partir de l'échantillon de sang ; et
(c) mesurer $C_1$ et mesurer $C_2$ dans le surnageant de l'échantillon.

**3.** Produit à injecter pour une utilisation de la revendication 1 ou de la revendication 2 dans lequel l'étape consistant à mesurer $C_1$ et l'étape consistant à mesurer $C_2$ est réalisée in vivo.

**4.** Appareil pour mesurer simultanément un volume de fluide extracellulaire (ECFV) et un volume de plasma vasculaire total (TVPV) d'un animal présentant une insuffisance rénale dans la revendication 1 comprenant :

(a) un moyen pour fournir le produit à injecter au système vasculaire de l'animal ;
(b) un moyen pour mesurer $C_1$ et $C_2$ in vivo dans le système vasculaire de l'animal ;
(c) un moyen pour calculer l'ECFV et le TVPV ;
(d) un moyen pour afficher les valeurs calculées de l'ECFV et du TVPV, et éventuellement
(e) un moyen pour calculer l'IFV et un moyen pour afficher la valeur calculée de l'IFV.

**5.** Appareil de la revendication 4 dans lequel l'appareil est incorporé dans un dispositif d'hémodialyse.

**6.** Produit à injecter ayant un volume V contenant une première molécule et une deuxième molécule, la première molécule (i) étant du dextrane ayant une taille moléculaire de 1 kDa à 20 kDa marqué avec un premier colorant fluorescent ayant une première longueur d'onde d'excitation et une première longueur d'onde d'émission, (ii) étant non métabolisée et perméable aux parois de vaisseaux du système vasculaire de l'animal et (iii) ayant une première intensité de fluorescence d'émission de $F_1$, et la deuxième molécule (i) étant du dextrane ayant une taille moléculaire de 70 kDa à 500 kDa marqué avec un deuxième colorant fluorescent ayant une deuxième longueur d'onde d'excitation et une deuxième longueur d'onde d'émission, (ii) étant non métabolisée et imperméable aux parois de vaisseaux du système vasculaire de l'animal et (iii) ayant une deuxième intensité de fluorescence d'émission de $F_2$, pour une utilisation dans un procédé pour mesurer simultanément un volume de fluide extracellulaire (ECFV) et un volume de plasma vasculaire total (TVPV) dans un animal présentant une insuffisance rénale, le procédé comprenant les étapes consistant à :

(a) administrer le produit à injecter dans le système vasculaire de l'animal ;
(b) laisser la première molécule et la deuxième molécule atteindre chacune une concentration d'état stable d'équilibre dans le système vasculaire de l'animal ;
(c) exciter la première molécule in vivo dans le système vasculaire de l'animal avec une première source de lumière d'excitation ayant une première longueur d'onde d'excitation et exciter la deuxième molécule in vivo dans le système vasculaire de l'animal avec une deuxième source de lumière d'excitation ayant une deuxième longueur d'onde d'excitation ;
(d) mesurer la troisième intensité de fluorescence d'émission $F_3$ issue de la première molécule in vivo dans le système vasculaire de l'animal et mesurer la quatrième intensité de fluorescence d'émission $F_4$ issue de la deuxième molécule in vivo dans le système vasculaire de l'animal ; et
(e) calculer l'ECFV en utilisant l'équation ECFV = $(F_1*V)/F_3$ et le TVPV en utilisant l'équation TVPV = $(F_2*V)/F_4$.

**7.** Produit à injecter pour une utilisation de la revendication 6, dans lequel le procédé comprend une étape supplémentaire de calcul du volume de fluide interstitiel (IFV) en utilisant l'équation IFV = ECFV - TVPV.

**8.** Produit à injecter pour une utilisation de l'une quelconque des revendications 1 à 3 ou 6 à 7, dans lequel l'administration est par injection intraveineuse, éventuellement dans lequel l'injection est une injection bolus ou une perfusion.

**9.** Produit à injecter pour une utilisation de l'une quelconque des revendications 1 à 3 ou 6 à 8, dans lequel le premier colorant fluorescent et le deuxième colorant fluorescent sont choisis dans le groupe constitué par : un colorant xanthène, CAL Flour, Alexa Flour, Oregon Green, une carbocyanine, la fluorescéine, l'isothiocyanate de fluorescéine (FITC), la carboxy-fluorescéine, une cyanine, une rhodamine, la tétraméthylrhodamine (Tamra), l'isothiocyanate de tétraméthylrhodamine (TRITC), l'isothiocyanate de rhodamine X (XRITC), Texas Red, DiLight et le vert d'indocyanine (ICG).

FIG. 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6355624 B
- US 12425827 B

**Non-patent literature cited in the description**

- **AGARWAL R. et al.** Diagnostic Utility of Blood Volume Monitoring in Hemodialysis Patients. *Am J of Kidney Diseases,* 2008, vol. 51, 242-254
- **RODRIGUEZ H. J. et al.** Assessment of Dry Weight by Monitoring Changes in Blood Volume During Hemodialysis using Crit-Line. *Kidney International,* 2005, vol. 68, 854-861
- **KRAEMER M. et al.** Detection Limit of Methods to Assess Fluid Status Changes in Dialysis Patients. *Kidney International,* 2006, vol. 69, 1609-1620
- **DASSELAAR J. J. et al.** Measurment of Relative Blood Volume Changes During Haemodialysis: Merits and Limitations. *Nephrol Dial Transplant,* 2005, vol. 20, 2043-2049
- **MITRA, S. et al.** Serial Determinations of Absolute Plasma Volume with Indocyanine Green During Hemodialyais. *J Am Soc of Nephrology,* 2003, vol. 14 (9), 2345-51
- **ZHU et al.** Segment-Specific Resistivity Improves Body Fluid Volume Estimates from Bioimpedence Spectroscopy in Hemodialysis Patients. *J Appl Physio,* 2006, vol. 100, 717-724
- **DE LORENZO A. et al.** Predicting Body Cell Mass With Bioimpedance by Using Theoretical Methods: a Technological Review. *J Appl Physiology,* 1997, vol. 82, 1542-1558
- **KUHLMANN, M. K. et al.** Bioimpedence, Dry Weight and Blood Pressure Control: New Methods and Consequences. *Current Opinion in Nephrology and Hypertension,* 2005, vol. 14, 543-549
- **ALTMAN P. L.** Blood and Other Body Fluids. *Fed of Am Societies for Experimental Biology,* 1961
- **YU W. et al.** Rapid Determinations of Renal Filtration Function using an Optical Ratiometric Image Approach. *Am J Physiology - Renal Physiology,* 2007, vol. 292 (6), F1873-80
- **DASSELAAR J. J. et al.** Measurement of Relative Blood Volume Changes During Haemodialysis: Merits and Limitations. *Nephrol Dial Transplant,* 2005, vol. 20, 2043-2049
- **ALTMAN P. L.** Blood and Other Body Fluids. *Fed of Am Societies for Experimental Biology,* 1961